# EUROPÄISCHE PATENTSCHRIFT

(11) **EP 2 190 443 B1**
(45) Veröffentlichungstag und Bekanntmachung des Hinweises auf die Patenterteilung: **20.04.2011**
(21) Anmeldenummer: 08785785.0
(22) Anmeldetag: 02.09.2008
(51) Int. Cl.: A61K 33/44, B01J 23/889, B01J 37/03, C01B 31/02

(54) **KOHLENSTOFFNANORÖHRCHENPULVER, KOHLENSTOFFNANORÖHRCHEN UND VERFAHREN ZU IHRER HERSTELLUNG**
CARBON NANOTUBE POWDER, CARBON NANOTUBE, AND METHOD FOR THE PRODUCTION THEREOF
POUDRE DE NANOTUBES DE CARBONE, NANOTUBES DE CARBONE ET PROCÉDÉ POUR LEUR PRODUCTION

(30) Priorität: 14.09.2007 DE 102007044031
(43) Veröffentlichungstag der Anmeldung: 02.06.2010
(73) Patentinhaber: Bayer MaterialScience AG, 51368 Leverkusen (DE)
(72) Erfinder: MEYER, Helmut, 51519 Odenthal (DE); HOCKE, Heiko, 51375 Leverkusen (DE); WEBER, Ralph, 42799 Leichlingen (DE); SCHMID, Martin, 51515 Kürten (DE); BRAMER-WEGER, Elmar, 53347 Alfter (DE); VOETZ, Matthias, 51375 Leverkusen (DE); MLECZKO, Leslaw, 41542 Dormagen (DE); RUDOLF, Reiner, 51373 Leverkusen (DE); WOLF, Aurel, 42489 Wülfrath (DE); BUCHHOLZ, Sigurd, 50767 Köln (DE)
(86) Internationale Anmeldenummer: PCT/EP2008/007138
(87) Internationale Veröffentlichungsnummer: WO 2009/036877

(56) Entgegenhaltungen:
- US-A1- 2003 224 168
- H.SHIOYAMA, T.AKITA: "A chemical route to carbon nanotubes" CARBON, Bd. 41, 2003, Seiten 179-181, XP002557582
- SHEILA F. BARGA: "Structure and Dynamics of carbon nanoscrolls" NANO LETTERS, Bd. 4, Nr. 5, 2004, Seiten 881-884, XP002557583
- R.RURALI: "prediction of giant electroactuation for papyruslike carbon nonoscroll structures" PHYSICAL REVIEW, Bd. B, Nr. 74, 2006, Seiten 085414-1-085414-4, XP002557584

## Beschreibung

Die Erfindung betrifft ein neues Kohlenstoffnanoröhrchenpulver, aufweisend Kohlenstoffnanoröhrchen, die eine rollenförmige Struktur aufweisen, ferner neue Kohlenstoffnanoröhrchen mit rollenförmiger Struktur, neue Verfahren zur Herstellung der Kohlenstoffnanoröhrchenpulver und der Kohlenstoffnanoröhrchen, und ihre jeweilige Verwendung. Die Kohlenstoffnanoröhrchen werden im Folgenden abkürzend als "CNT" bezeichnet.

Unter Kohlenstoffnanoröhrchen werden nach dem Stand der Technik hauptsächlich zylinderförmige Kohlenstoffröhren mit einem Durchmesser zwischen 3 und 100 nm verstanden und einer Länge, die ein Vielfaches des Durchmessers beträgt. Diese Röhrchen bestehen aus einer oder mehreren Lagen geordneter Kohlenstoffatome und weisen einen in der Morphologie unterschiedlichen Kern auf. Diese Kohlenstoffnanoröhrchen werden beispielsweise auch als "carbon fibrils" oder "hollow carbon fibres" bezeichnet.

In der Fachliteratur sind Kohlenstoffnanoröhrchen seit langem bekannt. Obwohl Iijima (Publikation: S. Iijima, Nature 354, 56-58, 1991) allgemein als Entdecker der Nanotubes bezeichnet wird, sind diese Materialien, insbesondere faserförmige Graphitmaterialien mit mehreren Graphitschichten, schon schon seit den 70er bzw. frühen 80er Jahren bekannt. Tates und Baker (GB 1469930A1, 1977 und EP 56004 A2, 1982) beschrieben erstmals die Abscheidung von sehr feinem faserförmigen Kohlenstoff aus der katalytischen Zersetzung von Kohlenwasserstoffen. Allerdings werden die auf Basis kurzkettiger Kohlenwasserstoffe hergestellten Kohlenstofffilamente nicht näher im Bezug auf ihren Durchmesser charakterisiert.

Die Herstellung von Kohlenstoffnanoröhrchen mit Durchmessern kleiner 100 nm ist erstmals in EP 205 556 B1 bzw. WO A 86/03455 beschrieben. Für die Herstellung werden hier leichte (d. h. kurz- und mittelkettige aliphatische oder ein- oder zweikernige aromatische) Kohlenwasserstoffe und ein auf Eisen basierender Katalysator eingesetzt, an dem Kohlenstoffträgerverbindungen bei einer Temperatur oberhalb von 800-900°C zersetzt werden.

Die heute bekannten Methoden zur Herstellung von Carbon Nanotubes umfassen Lichtbogen-, Laserablations- und katalytische Verfahren. Bei vielen dieser Verfahren werden Ruß, amorpher Kohlenstoff und Fasern mit hohen Durchmessern als Nebenprodukte gebildet. Bei den katalytischen Verfahren kann zwischen der Abscheidung an geträgerten Katalysatorpartikeln und der Abscheidung an in-situ gebildeten Metallzentren mit Durchmessern im Nanometerbereich (sogenannte Flow-Verfahren) unterschieden werden. Bei der Herstellung über die katalytische Abscheidung von Kohlenstoff aus bei Reaktionsbedingungen gasförmigen Kohlenwasserstoffen (im folgenden CCVD; Catalytic Carbon Vapour Deposition) werden als mögliche Kohlenstoffspender Acetylen, Methan, Ethan, Ethylen, Butan, Buten, Butadien, Benzol und weitere, Kohlenstoff enthaltende Edukte genannt.

Die Katalysatoren beinhalten in der Regel Metalle, Metalloxide oder zersetzbare bzw. reduzierbare Metallkomponenten. Beispielsweise sind im Stand der Technik als Metalle Fe, Mo, Ni, V, Mn, Sn, Co, Cu und weitere genannt. Die einzelnen Metalle haben meist zwar eine Tendenz, Nanotubes zu bilden, allerdings werden laut Stand der Technik hohe Ausbeuten und geringe Anteile amorpher Kohlenstoffe vorteilhaft mit Metallkatalysatoren erreicht, die eine Kombination der oben genannten Metalle enthalten.

Besonders vorteilhafte Systeme basieren gemäß dem Stand der Technik auf Kombinationen, die Fe oder Ni enthalten. Die Bildung von Kohlenstoffnanoröhrchen und die Eigenschaften der gebildeten Röhrchen hängen in komplexer Weise von der als Katalysator verwendeten Metallkomponente oder einer Kombination mehrerer Metallkomponenten, dem verwendeten Trägermaterial und der Wechselwirkung zwischen Katalysator und Träger, dem Eduktgas und -partialdruck, einer Beimischung von Wasserstoff oder weiteren Gasen, der Reaktionstemperatur und der Verweilzeit bzw. dem verwendeten Reaktor ab. Eine Optimierung stellt eine besondere Herausforderung für einen technischen Prozess dar.

Anzumerken ist, dass die bei der CCVD verwendete und als Katalysator bezeichnete Metallkomponente im Laufe des Syntheseprozesses verbraucht wird. Dieser Verbrauch ist auf eine Desaktivierung der Metallkomponente zurückzuführen, z. B. aufgrund von Abscheidung von Kohlenstoff auf dem gesamten Partikel, die zur vollständigen Bedeckung des Partikels führt (dem Fachmann ist dies als "Encapping" bekannt). Eine Reaktivierung ist in der Regel nicht möglich bzw. wirtschaftlich nicht sinnvoll. Es werden oftmals nur maximal wenige Gramm Kohlenstoffnanoröhrchen pro Gramm Katalysator erhalten, wobei der Katalysator hier die verwendete Gesamtheit von Träger und Katalysator umfasst. Aufgrund des geschilderten Verbrauchs an Katalysator stellt eine hohe Ausbeute an Kohlenstoffnanoröhrchen bezogen auf den eingesetzten Katalysator eine wesentliche Anforderung an Katalysator und Verfahren dar.

Für eine technische Herstellung von Kohlenstoffnanoröhrchen z. B. als Bestandteil zur Verbesserung der mechanischen Eigenschaften oder Leitfähigkeit von Kompositmaterialien ist wie bei allen technischen Verfahren eine hohe Raum-Zeit-Ausbeute bei Erhaltung der besonderen Eigenschaften der Nanoröhrchen sowie Minimierung der aufzuwendenden Energie und Betriebsstoffe anzustreben. Auf der Laserablation von Kohlenstoff basierende Anwendungen liefern oftmals nur geringe Produktionsraten und hohe Anteile an amorphem Kohlenstoff bzw. Ruß. Die Überführung dieser Aufbauten im Labormaßstab mit Produktionsraten weniger Gramm pro Tag in einen technischen Maßstab ist meist nur schwer möglich. So ist auch die Laser-Ablation teuer und ein Scale-Up schwierig. Auch verschiedene in der Literatur beschriebene Verfahren zur Herstellung von Kohlenstoffnanoröhrchen durch CCVD zeigen zwar die prinzipielle Eignung verschiedener Katalysatoren, weisen oftmals aber nur eine geringe Produktivität auf.

In der Patentliteratur sind zur Herstellung von Kohlenstoffnanoröhrchen verschiedene Verfahren und Katalysatoren bekannt. Bereits in EP 0205 556 A 1 (Hyperion Catalysis International) werden solche Kohlenstoffnanoröhrchen beschrieben, die über einen eisenhaltigen Katalysator und die Umsetzung verschiedener Kohlenwasserstoffe bei hohen Temperaturen oberhalb von 800-1000°C hergestellt werden. Von Shaikhutdinov et al. (Shamil' K. Shaikhutdinov, L.B. Avdeeva, O.V. Goncharova, D.I. Kochubey, B.N. Novgorodov, L.M. Plyasova, "Coprecipitated Ni-Al and Ni-Cu-Al catalysts for methane decomposition and carbon deposition I.", Applied Catalysis A: General, 126, 1995, Seiten 125-139) werden Ni-basierende Systeme als aktiv in der Zersetzung von Methan zu Kohlenstoffnanomaterialien genannt.

In CA 2374848 (Centre National de la Recherche Scientifique, FR) wird als möglicher Prozess für die Massenproduktion von Carbon Nanotubes ein Verfahren dargelegt, bei dem mit Acetylen als Kohlenstoffdonator an einem Kobalt-Katalysator eine Ausbeute von 3 g CNTs / g Katalysator erzielt wird. Diese vergleichsweise sehr geringe Ausbeute lässt den Prozess hinsichtlich der Gewährleistung einer guten Durchmischung unkritisch erscheinen, macht allerdings aufwendige Aufreinigungsschritte erforderlich, um ein für die Anwendung geeignetes Produkt zu erhalten.

Ebenfalls nur sehr geringe Ausbeuten (max. 0,35 g CNTs / g Katalysator) erzielen Mauron et al. (Ph. Mauron, Ch. Emmenegger, P. Sudan, P. Wenger, S. Rentsch, A. Züttel, "Fluidised-bed CVD synthesis of carbon nanotubes on Fe2O3/MgO", Diamond and Related Materials 12 (2003) 780-785) bei der Herstellung von CNTs aus Iso-Pentan bzw. Acetylen an einem Eisen-Katalysator. Sie gehen daher auch nicht weiter auf eventuelle Schwierigkeiten bei der Durchmischung im Reaktor während des Wachstumsprozesses der Agglomerate ein.

EP 1399384 (Institut National Polytechnique, Toulouse, FR) beschreibt die Herstellung von Carbon Nanotubes in einem CCVD-Prozess mit vorgeschaltetem Reaktor zur Inline-Katalysatorherstellung, wobei der Katalysator eine mittlere Partikelgröße zwischen 10 µm und 1000 µm aufweisen kann und einen Volumenzuwachs der Agglomerate bis zum Zwanzigfachen der Katalysatormenge erreichen kann. Bezüglich Fluidisierung wird dort lediglich gefordert, dass die Gasleerrohrgeschwindigkeit im Reaktor oberhalb der Mindestfluidisierungsgeschwindigkeit des Partikelkollektivs im Reaktor und unterhalb der für die Ausbildung einer Kolbenströmung erforderlichen Gasgeschwindigkeit bleibt.

In einer Dissertation von Nijkamp (Universiteit Utrecht / NL, 2002, "Hydrogen Storage using Physisorption Modified Carbon Nanofibers and Related Materials") wird die Herstellung von Carbon Nanotubes mittels nickelhaltiger Katalysatoren und Methan als Kohlenstoffdonator beschrieben. Dabei wird allerdings nur der Labormaßstab betrachtet (Reaktorinnendurchmesser 25 mm) und bei einer relativ niedrigen Ausbeute (27 g CNTs / g Katalysator) insgesamt nur sehr wenig Material (10-30 g) erzeugt. Das so erzeugte Material muss vor der Weiterverwendung aufgereinigt werden, da die Katalysatorreste störend auf die meisten Anwendungen wirken und Nickel aufgrund seiner krebserregenden Wirkung nicht in die Endprodukte gelangen darf.

In den bis hier genannten unterschiedlichen Verfahren unter Einsatz verschiedener Katalysatorsysteme werden Kohlenstoffnanoröhrchen verschiedener Strukturen hergestellt, die aus dem Prozess überwiegend als Kohlenstoffnanoröhrchenpulver entnommen werden können.

Übliche Strukturen solcher Röhrchen sind solche vom Zylinder Typ. Bei den zylindrischen Strukturen unterscheidet man zwischen den einwandigen Monokohlenstoffnanoröhrchen (Single Wall Carbon Nano Tubes) und den mehrwandigen zylindrischen Kohlenstoffnanoröhrchen (Multi Wall Carbon Nano Tubes). Gängige Verfahren zu ihrer Herstellung sind z.B. Lichtbogenverfahren (arc discharge), Laser Ablation (laser ablation), Chemische Abscheidung aus der Dampfphase (CVD process) und Katalytisch Chemische Abscheidung aus der Dampfphase (CCVD process) .

Nach einem Lichtbogenverfahren können ebenfalls derartige zylindrische Kohlenstoffröhrchen hergestellt werden. Iijima, Nature 354, 1991, 56-8, berichtet über die Bildung von Kohlenstoffröhrchen im Lichtbogenverfahren, die aus 2 oder mehr Graphenlagen bestehen, die zu einem nahtlos geschlossen Zylinder aufgerollt und ineinander geschachtelt sind. Abhängig vom Aufrollvektor sind chirale und achirale Anordnungen der Kohlenstoffatome längs der Längsachse der Kohlenstofffaser möglich.

Die WO86/03455A1, beschreibt die Herstellung von Kohlenstofffilamenten, die eine zylindrische Struktur mit einem konstanten Durchmesser von 3.5 bis 70 nm aufweisen, einem Aspektverhältnis (Verhältnis von Länge zu Durchmesser) von größer 100 und einer Kernregion. Diese Fibrilen bestehen aus vielen, durchgängigen Lagen geordneter Kohlenstoffatome, die konzentrisch um die zylindrische Achse der Fibrilen angeordnet sind. Diese zylinderartigen Nanotubes wurden nach einem CVD Prozess aus kohlenstoffhaltigen Verbindungen mittels eines metallhaltigen Partikels bei einer Temperatur zwischen 850°C und 1200 °C hergestellt.

Aus der WO2007/093337A2 ist noch ein Verfahren zur Herstellung eines Katalysators bekannt geworden, der für die Herstellung von konventionellen Carbon Nanotubes mit zylindrischer Struktur geeignet ist. Bei Verwendung dieses Katalysators in einem Festbett werden höhere Ausbeuten von zylindrischen Kohlenstoffnanoröhrchen mit einem Durchmesser im Bereich von 5 bis 30 nm gewonnen.

Ein völlig anderer Weg zur Herstellung zylindrischer Kohlenstoffnanoröhrchen wurde von Oberlin, Endo und Koyam beschrieben (Carbon 14, 1976, 133). Dabei werden aromatische Kohlenwasserstoffe, z.B. Benzen, an einem Metallkatalysator umgesetzt. Die entstandene Kohlenstoffröhre zeigt einen gut definierten, graphitischen hohlen Kern der ungefähr den Durchmesser des Katalysatorpartikels hat, auf dem sich weiterer weniger graphitisch geordneter Kohlenstoff befindet. Die Autoren vermuten, dass zunächst der graphitische Kern durch schnelles, katalytisches Wachstum gebildet wird und sich danach weiterer Kohlenstoff pyrolytisch abscheidet. Die gesamte Röhre kann durch Behandlung bei hoher Temperatur (2500°C - 3000°C) graphitisiert werden.

Die meisten der oben genannten Verfahren (mit Lichtbogen, Sprühpyrolyse bzw. CVD) werden heute zur Herstellung von Carbon Nanotubes genutzt. Die Herstellung einwandiger zylindrischer Kohlenstoffnanoröhrchen ist jedoch apparativ sehr aufwendig und verläuft nach den bekannten Verfahren mit sehr geringer Bildungsgeschwindigkeit und oft auch mit vielen Nebenreaktionen, die zu einem hohen Anteil an unerwünschten Verunreinigungen führen, d.h. die Ausbeute solcher Verfahren ist vergleichsweise gering. Deshalb ist die Herstellung derartiger Carbon Nanotubes auch heute noch extrem kostspielig und sie kommen nur für hoch spezialisierte Anwendungen in geringen Mengen zum Einsatz.

Bacon et al., J. Appl. Phys. 34, 1960, 283-90, beschreibt bereits 1960 die Existenz von Kohlenstoff -Whiskern, die aus einer aufgerollten Graphenlage bestehen. Die Struktur wird als Scroll Type bezeichnet. Das von Bacon beschriebene Herstellverfahren beruht auf Verdampfen einer Kohlenstoffelektrode im Lichtbogen (arc discharge).

Ähnliche Strukturen von Kohlenstoffröhrchen, bei denen eine zusammenhängende Graphenlage (sogenannter scroll type) oder unterbrochene Graphenlage (sogenannter onion type) die Basis für den Aufbau der Nanoröhre ist, wurden später auch von Zhou et al., Science, 263, 1994, 1744-47 und von Lavin et al., Carbon 40, 2002, 1123-30 gefunden. Diese Kohlenstoffnanoröhrchen liegen in einem nach dem Lichtbogenverfahren produzierten Ruß im Gemisch neben vielen weiteren Kohlenstoffstrukturen vor. Eine Separierung oder Reindarstellung solcher Scroll-type oder Oniontype-Kohlenstoffnanoröhrchen ist ohne weiteres nicht möglich. Eine industrielle Produktion solch spezieller Formen kommt daher nicht in Betracht.

Kohlenstoffnanoröhrchen, die aus einer einzelnen aufgerollten Graphenlage bestehen, wurden später auch mittels eines Pyrolyseverfahrens von Ruland et al., Carbon 41, 2003, 423-27) hergestellt.Dravid et al., Science 259, 1993, 1601-04, und Feng et al., J. Phys. Chem. Solids, 58, 1997, 1887-92, beschreiben intermediäre Strukturen, bei denen Graphenlagen um ein einzelnes dickeres Kohlenstoffnanoröhrchen vom sogenannten Bucky type gewunden sind. Bucky type ist eine Bezeichnung für mehrwandige Kohlenstoffnanoröhrchen mit runden geschlossenen Enden aus Graphit, die konzentrische geschlossene Graphitzylinder aufweisen.

Bei all diesen Verfahren zur Herstellung von Scroll- oder Zwiebel-artigen Kohlenstoffröhrchen ist der Energieaufwand sehr hoch und die Ausbeute gering, was eine praktikable oder industrielle Herstellung unmöglich macht.

Die Herstellung von mehrwandigen Carbon Nanotubes, in Form von ineinander geschachtelten nahtlosen zylindrischen Nanotubes erfolgt heute kommerziell in größeren Mengen überwiegend unter Verwendung katalytischer Verfahren. Diese Verfahren zeigen üblicherweise eine höhere Ausbeute als die oben genannten Lichtbogen- und andere Verfahren und werden heute typischerweise im kg-Maßstab (einige hundert kilo/Tag weltweit) durchgeführt. Die so hergestellten MW-Carbon Nanotubes sind in der Regel um einiges kostengünstiger als die einwandigen Nanotubes und werden deshalb in gewissen Mengen als leistungsteigerndes Additiv in anderen Werkstoffen eingesetzt.

Aufgabe der vorliegenden Erfindung ist es, ein Verfahren zu entwickeln, um Kohlenstoffnanoröhrchen in noch größeren Mengen herzustellen, die mindestens die Eigenschaften der bekannten MWCNT-Strukturen aufweisen.

Des weiteren soll das CNT-Material eine hohe Reinheit in Bezug auf metallische Verunreinigungen und amorphen Kohlenstoff aufweisen, Verunreinigungen, die zu einer Verschlechterung von Materialeigenschaften bei der Einarbeitung in Matrixwerkstoffe z.B. in Polymere führen können. Weiterhin soll das Produkt insbesondere eine gute Rieselfähigkeit und besonders eine weitgehende Staubfreiheit sowie ein möglichst hohes Schüttgewicht der CNT aufweisen, um Transport und Handhabung sowohl bei der Herstellung, als auch beim Ab- oder Umfüllen des CNT-Materials und der späteren Einarbeitung zu erleichtern. Insbesondere wünschenswert wäre noch eine hohe innere Oberfläche des CNT-Materials

Diese Aufgabe konnte durch die Bereitstellung eines ausgewählten katalytischen Gasphasenverfahrens gelöst werden, durch das unter Auswahl spezieller geeigneter Katalysatoren und Verfahrensbedingungen neuartige Kohlenstoffnanoröhrchenpulver gebildet werden, die zum überwiegenden Teil aus Kohlenstoffnanoröhrchen bestehen, welche aus einer oder mehreren durchgehenden Graphitschichten bestehen, die zu einer röhrenförmigen Struktur aufgerollt sind.

Gegenstand der Erfindung ist somit ein Kohlenstoffnanoröhrchenpulver, aufweisend Kohlenstoffnanoröhrchen, dadurch gekennzeichnet, dass die Kohlenstoffnanoröhrchen im wesentlichen aus einer oder mehreren Graphitschichten bestehen, wobei die Graphitschichten aus zwei oder mehr übereinander angeordneten Graphenlagen aufgebaut sind, und die Graphitschichten eine gerollte Struktur bilden, dass die Kohlenstoffnanoröhrchen im Querschnitt eine spiralförmige Anordnung der Graphitschichten aufweisen, wobei der mittlere Durchmesser der Kohlenstoffnanoröhrchen 3 bis 100 nm, bevorzugt 4 bis 75 nm, besonders bevorzugt 5 bis 30 nm beträgt.

Im Unterschied zu den bisherigen in der Literatur beschriebenen CNT mit Strukturen vom Scrolltyp mit nur einer durchgehenden oder unterbrochenen Graphenlage sind bei diesen neuen Strukturformen des Kohlenstoffs mehrere Graphenlagen zu einem Stapel zusammengefasst, der aufgerollt vorliegt (Multiscroll Type). Die erfindungsgemäßen Kohlenstoffnanoröhrchen können damit als weitere Kohlenstoffform betrachtet werden und sind strukturell vergleichbar zu den bekannten Kohlenstoffnanoröhrchen vom einfachen Scroll Typ wie mehrwandinge zylindrische Monokohlenstoffnanoröhrchen (cylindrical SWCNT) zu einwandingen zylindrischen Kohlenstoffnanoröhrchen (cylindrical MWCNT).

Anders als bei den im Stand der Technik noch vereinzelt beschriebenen zwiebelartigenStrukturen (onion type structure) verlaufen die einzelnen Graphen- bzw. Graphitschichten in den neuen Kohlenstoffnanoröhrchen, wie erste Untersuchungen zeigen, im Querschnitt gesehen offenbar durchgehend vom Zentrum der CNT bis zum äußeren Rand ohne Unterbrechung. Dies kann z. B. eine verbesserte und schnellere Interkalierung anderer Materialien, wie Lithium-, Kalium-, Rubidiummetall oder Eisenchlorid, im Röhrchengerüst ermöglichen, da mehr offene Ränder als Eintrittszone der Interkalate zur Verfügung stehen im Vergleich zu CNTs mit einfacher Scrollstruktur (Carbon 34, 1996, 1301-3) oder CNTs mit zwiebel-artiger Scrollstruktur (Science 263, 1994, 1744-7).

Die Kohlenstoffnanoröhrchen liegen in dem Kohlenstoffnanoröhrchenpulver typischerweise insbesondere als Agglomerate langer Fasern vor.

Bevorzugt ist ein Kohlenstoffnanoröhrchenpulver enthaltend Kohlenstoffnanoröhrchen, bei dem das Länge zu Durchmesser-Verhältnis der Kohlenstoffnanoröhrchen mindestens 5, insbesondere mindestens 30, besonders bevorzugt mindestens 50, beträgt.

Ein großer Vorteil des erfindungsgemäßen Kohlenstoffnanoröhrchenpulvers ist seine weitgehende Staubfreiheit und gute Rieselfähigkeit sowie sein hohes Schüttgewicht bei gleichzeitig hoher innerer Oberfläche. Diese sorgen für eine problemlose Handhabung der neuen Kohlenstoffnanoröhrchenpulver beim Umgang mit dem Material und seiner Einarbeitung in andere Werkstoffe, z.B. Kunststoffe. Die neuen Multiscroll-Kohlenstoffnanoröhrchen, die aufgrund ihrer Struktur an den äußeren freien Seiten der Graphenlagen offenbar leicht einer chemischen Funktionalisierung zugänglich sind, erlauben bei Verwendung als Polymeradditiv z.B. eine verbesserte Anbindung an den jeweiligen Matrixwerkstoff.

In derartigen neuen Kohlenstoffnanoröhrchen stehen z.B. deutlich mehr reaktive Kohlenstoffatome an den äußeren Rändern entlang der Achse der Kohlenstoffnanoröhrchen zur Verfügung als bei bekannten Monoscrollröhrchen oder den Zylinder-artigen Kohlenstoffröhrchen. Dadurch können grundsätzlich mehr funktionelle Gruppen und diese zudem leichter aufgebracht werden als bei anderen Kohlenstoffnanoröhrchen, was z.B. für eine verbesserte Anbindung der Röhrchen über diese Gruppen in Polymeren, zur besseren Stabilisierung der Röhrchen in Lösungsmitteln oder für die Beladung mit pharmazeutischen Wirkstoffen oder aktiven Katalysatorkomponenten von Bedeutung ist. Im Gegensatz insbesondere zu den zylindrischen Kohlenstoffnanoröhrchen mit im Querschnitt gesehen kreisförmig geschlossenen Graphenlagen wird bei der chemischen Funktionalisierung die eigentliche Struktur der neuen Röhrchen nicht oder nur wenig beeinflusst, so dass die elektrischen Eigenschaften weitestgehend erhalten bleiben bzw. sich nicht oder nur minimal verschlechtern.

Bevorzugt ist ein Kohlenstoffnanoröhrchenpulver, das dadurch gekennzeichnet ist, dass der Gehalt des Pulvers an Verunreinigungen, insbesondere von Metallen oder Metallverbindungen, insbesondere bevorzugt Metalloxiden, höchstens 7 Gew.-%, bevorzugt höchstens 5 Gew.-% beträgt.

Die Verunreinigungen umfassen insbesondere Metalle oder Metallverbindungen der Übergangsmetalle, insbesondere der VII. und VIII. Nebengruppe des Periodensystems der Elemente, oder der Alkalimetalle oder der Erdalkalimetalle oder Silizium oder Siliziumoxid, und insbesondere bevorzugt Metalle oder Metallverbindungen ausgewählt aus der Gruppe Aluminium, Magnesium, Titan, Zirkon, Natrium, Kalzium, Eisen, Nickel, Mangan, Molybdän und Kobalt. In Anwendungen der CNT, die elektronische (Halbleiter-)Bauelemente oder Brennstoffzellen oder Batterien betreffen ist die Anwesenheit von Fremdmetallen generell unerwünscht. Einige der vorgenannten Metalle gelten zudem als toxikologisch bedenklich und sind wie eingangs dargestellt bei verschiedenen aus dem Stand der Technik bekannten CNT herstellungsbedingt vorzufinden. Bei Verwendung der CNT als Additiv für Polymere ist das Vorliegen bestimmter Metalle (z.B. Eisen in Polycarbonat) ebenfalls unerwünscht, da sie den Abbau der Polymere unter Umständen katalytisch beschleunigen und die Lebensdauer entsprechender Komposite herabsetzen. Mit dem besonderen neuen Kohlenstoffnanoröhrchenpulver wird dieser Nachteil vermieden.

Der Gehalt des Kohlenstoffnanoröhrchenpulvers an pyrolytisch abgeschiedenem Kohlenstoff beträgt bevorzugt höchstens 7 Gew.-%, besonders bevorzugt höchstens 2 Gew.-%, ganz besonders bevorzugt höchstens 1 Gew.-%. Pyrolytisch abgeschiedener Kohlenstoff liegt vorwiegend als amorpher Kohlenstoff vor, weist keine Kristallstruktur oder eine die nicht mit der typischen geordneten graphitischen Kristallstruktur der reinen CNT vergleichbar ist auf. Pyrolytisch abgeschiedener Kohlenstoff enthält gegebenenfalls Verunreinigungen höherer, mehrkerniger Aromaten auf die technisch und ökologisch unerwünscht sind und müsste aufwendig von den reinen auf Graphitstruktur basierenden Fasern abgetrennt werden, um die Werkstoffeigenschaften von aus CNT gefertigten Kompositen nicht nachteilig zu beeinflussen.

In einer bevorzugten Ausgestaltung der Erfmdung liegt das Kohlenstoffnanoröhrchenpulver als Agglomerat vor, wobei mindestens 95 Gew.-% der Agglomeratteilchen einen Außendurchmesser im Bereich von 5 µm bis 10.000 µm, bevorzugt 30 µm bis 5.000 µm, besonders bevorzugt 50 µm bis 3.000 µm haben. Dies gewährleistet eine gute Fließfähigkeit bzw. Rieselfähigkeit des CNT-Pulvers, so dass die Handhabung des Produktes bei Dosierung, Umfüllung, Lagerung und anderen Prozessschritten bei Produktion, Lagerung Veredelung, Einarbeitung und anderen für das Produkt relevanten Verarbeitungschritten wesentlich vereinfacht wird. Gleichzeitig wird das Staubungsverhalten stark herabgesetzt. Dies vereinfacht die technischen und organisatorischen Maßnahmen zur Minimierung der Staubbelastung am Arbeitsplatz und minimiert die Verschmutzung des Arbeitsplatzes.

Das Kohlenstoffnanoröhrchenpulver weist bevorzugt mehr als 50 %, besonders bevorzugt mindestens 90 %, ganz besonders bevorzugt mindestens 95 % der oben beschriebenen neuen Kohlenstoffnanoröhrchen mit rollenförmigen Graphenlagen auf.

Bevorzugt ist auch ein Kohlenstoffnanoröhrchenpulver, das dadurch gekennzeichnet ist, dass die Schüttdichte (gemäß EN ISO 60) des Kohlenstoffnanoröhrchenpulvers 20 bis 450 kg/m³, bevorzugt 80 bis 350 kg/m³, ganz besonders bevorzugt 110 bis 250 kg/m³ beträgt. Diese Agglomerateigenschaften einer für Kohlenstoffnanoröhrchenpulver vergleichsweise hohen Schüttdichte tragen wesentlich zu einer weitgehenden Staubfreiheit und einer guten Handhabung sowie einer platzsparenden und damit ökonomisch transportierbaren Abpackung bei. Zudem wird die Riesel- bzw. Fließfähigkeit positiv beeinflusst, da die Dichte neben anderen Eigenschaften wie z.B. Partikelform und Partikelgeometrie einen wesentlichen Einfluss auf diesen Parameter hat. Insbesondere bevorzugt ist ein Kohlenstoffnanoröhrchenpulver, das dadurch gekennzeichnet ist, dass die spezifische Oberfläche (Stickstoffadsorption gemäß BET) 20 bis 1500 m²/g, bevorzugt 30 bis 800 m²/g besonders bevorzugt 90 bis 600 m²/g beträgt. Durch eine große Oberfläche wird eine gute Adhäsion der Kohlenstoffnanoröhrchen z.B. in einemPolymerkomposit ermöglicht, wodurch bessere mechanische Eigenschaften erzielt werden können. Gleichzeitig steigt jedoch die Viskosität des Komposites erheblich an. Unter Berücksichtigung der Verfahren zur Einarbeitung der Kohlenstoffnanoröhrchenpulver bzw. Kohlenstoffnanoröhrchen in Materialien wie Polymere (z.B. durch Nutzung eines Extruders) und der benötigten Konzentration an Kohlenstoffnanoröhrchen ergibt sich der oben angegebene optimale BET Bereich.

Besonders bevorzugt ist auch ein Kohlenstoffnanoröhrchenpulver, in dem die Kohlenstoffnanoröhrchen im Querschnitt gesehen einen maximalen Außendurchmesser von bis zu 500 nm, vorzugsweise bis 100 nm, besonders bevorzugt bis 50 nm aufweisen und damit sehr einheitliche Kohlenstoffnanoröhrchenpulver mit einer engen Durchmesserverteilung der enthaltenen Kohlenstoffnanoröhrchen bilden.

Das erfindungsgemäße Kohlenstoffnanoröhrchenpulver ist zugänglich über ein spezielles katalytisches Gasphasenverfahren unter definierten Verfahrensbedingungen und den Einsatz ausgewählter Katalysatoren. Überraschenderweise wird bei der Anwendung eines Wirbelbettes anstelle eines Festbetts in Verbindung mit ausgewählten Katalysatoren für die Herstellung von Kohlenstoffnanoröhrchen, die auf Mangan und Kobalt basieren, sowie der Anwendung einer vergleichsweise kurzen Verweilzeit von Katalysator und Kohlenstoffnanoröhrchen im Wirbelbett ein Kohlenstoffnanoröhrchenpulver gewonnen, das sowohl Kohlenstoffnanoröhrchen mit einer neuen eigenen Struktur aufweist als auch die beschriebenen Verarbeitungsvorteile hat.

Weiterer Gegenstand der Erfindung ist ein Verfahren zur Herstellung eines Kohlenstoffnanoröhrchenpulvers, dadurch gekennzeichnet, dass C₁ - C₃-Kohlenwasserstoffe an einem heterogenen Katalysator bei einer Temperatur von 500 bis 1000°, bevorzugt 600 bis 800°C in einem Reaktor mit bewegtem Bett zersetzt werden, wobei die mittlere Verweilzeit des Katalysators in der Reaktionszone höchstens eine Stunde beträgt. Der dabei verwendete Katalysator ist ein Übergangsmetallkatalysator basierend auf Co und Mn, insbesondere deren Mischoxiden, wobei bevorzugt der Anteil von Co 40 bis 60 mol.-% und der von Mn 60 bis 40 mol.-% bezogen auf die Summe von Co und Mn beträgt. Als Kohlenwasserstoff kommen bevorzugt Ethen oder Propenin Frage, die einzeln oder als Gemisch auch zusammen mit Inertgasen (z.B. Stickstoff oder Edelgasen) dem Prozess zugeführt werden. Die mittlere Verweilzeit des Katalysators in der Reaktionszone beträgt bevorzugt 20 bis 45 min, besonders bevorzugt 25 bis 35 min.

Besonders bevorzugt wird der Prozess unter Verwendung von Ethen durchgeführt. Das Verfahren kann kontinuierlich, halbkontinuierlich oder diskontinuierlich durchgeführt werden, Bevorzugt kontinuierlich.

Der einzusetzende Katalysator wird einer bevorzugten Ausführungsform in einer Cofällung der katalytisch aktiven Metallverbindungen Co und Mn zusammen mit mindestens einer weiteren Komponente, die in weiteren Schritten der Katalysatorbehandlung entweder ein intermediäres Bindermaterial oder eine katalytisch aktive Mischverbindung formt, aus in Wasser löslichen Salzen in wässrigem Medium mittels Laugen hergestellt. Als Beispiele für derartige weitere Komponenten seien Al, Mg, Si, Zr, Ti, usw. bzw. dem Fachmann bekannten gängigen Mischmetalloxid-bildende Elemente genannt. Die Fällung wird insbesondere durch Zugabe alkalischer Lösungen, insbesondere von Alkalilauge zur Metallsalzlösung bewirkt. Der Gehalt der weiteren Komponenten kann bis zu 80 Gew.-% bezogen auf die gesamte Katalysatormasse betragen. Bevorzugt besitzen die Katalysatoren einen Anteil an weiteren Komponenten von 5-75 Gew.-%.

Der in Form eines Feststoffs anfallende Katalysator kann nach dem Fachmann bekannten Methoden wie z. B. Filtrieren, Zentrifugieren, Eindampfen und Einengen von den Eduktlösungen getrennt werden. Bevorzugt sind die Zentrifugation und die Filtration. Der erhaltene Feststoff kann weiter gewaschen oder direkt, wie erhalten, weiter eingesetzt werden. Für eine verbesserte Handhabbarkeit des erhaltenen Katalysators wird dieser bevorzugt getrocknet und gemahlen. Wie bei heterogenen Katalysatoren bekannt, kann eine weitere Konditionierung der Katalysatoren von Vorteil sein. Diese Konditionierung kann die Kalzinierung und thermische Behandlung sowie die Behandlung mit reaktiven Atmosphären oder z. B. Wasserdampf mit dem Ziel der Verbesserung der katalytischen Eigenschaften sein. Diese erzielt man z.B. über eine thermische Vorbehandlung in oxidierender Atmosphäre bei Temperaturen zwischen 300°C und 900°C.

Der Konditionierung vor- oder nachgeschaltet kann zudem eine Formgebung und/oder Klassierung sein. Danach ist der Katalysator direkt einsetzbar. Bevorzugt wird Katalysator mit einer Teilchengröße im Bereich von 30 bis 100 µm besonders bevorzugt von 40 bis 80 µm für die Durchführung des neuen Verfahrens eingesetzt. Hierdurch wird die Fluidisierung in der Wirbelschicht und die Ausbeute an Kohlenstoffnanoröhrchenpulver verbessert.

Der nach dem erfindungsgemäßen Verfahren besonders bevorzugt einzusetzende Katalysator enthält 45-55 mol.-% Mn und 55-45 mol.-% Co bezogen auf den Gehalt aktiver Komponenten in metallischer Form.

Das erfindungsgemäße Verfahren kann in verschiedenen Typen von Wirbelbettreaktoren mit guter Durchmischung des Reaktorinhalts durchgeführt werden. Beispielhaft seien hier insbesondere Reaktoren mit einem blasenbildenden, turbulenten oder durchstrahlten Wirbelbett, oder einem intern oder extern zirkulierenden Wirbelbett genannt. Besonders bevorzugt ist die Anwendung eines blasenbildenden Wirbelbetts. Es ist auch möglich, den Katalysator in einen mit Partikeln gefüllten Wirbelbettreaktor zu geben. Diese Partikel können Inertpartikel sein und/oder ganz oder teilweise aus einem weiteren katalytisch aktiven Material bestehen. Diese Partikel können auch Agglomerate von Kohlenstoffnanoröhrchen sein.

Das Verfahren lässt sich damit beispielsweise kontinuierlich oder diskontinuierlich durchführen, wobei sich kontinuierlich oder diskontinuierlich sowohl auf die Zufuhr des Katalysators als auch die Abfuhr der gebildeten Kohlenstoffnanoröhrchen mit dem verbrauchten Katalysator bezieht.

Als Eduktgase kommen Kohlenwasserstoffe aus der Reihe: Methan, Ethan, Propan, Ethen und Propen in Betracht. Es können auch Gemische der oben genannten Kohlenwasserstoffe eingesetzt werden.

Gegenstand der Erfindung sind auch Kohlenstoffnanoröhrchen, dadurch gekennzeichnet, dass die Kohlenstoffnanoröhrchen im wesentlichen aus einer oder mehreren Graphitschichten bestehen, wobei die Graphitschichten aus zwei oder mehr übereinander angeordneten Graphenschichten aufgebaut sind, und die Graphitschichten eine gerollte Struktur bilden, dass die Kohlenstoffnanoröhrchen im Querschnitt eine spiralförmig Anordnung der Graphitschichten aufweisen wobei der mittlere Durchmesser der Kohlenstoffnanoröhrchen 3 bis 100 nm, bevorzugt 4 bis 75 nm, besonders bevorzugt 5 bis 30 nm beträgt.

Das Länge zu Durchmesser-Verhältnis der Kohlenstoffnanoröhrchen beträgt insbesondere mindestens 5, bevorzugt mindestens 10, insbesondere bevorzugt mindestens 20, ganz besonders bevorzugt mindestens 50.

Der Gehalt der Kohlenstoffnanoröhrchen an Verunreinigungen insbesondere von Metallen oder Metallverbindungen, insbesondere bevorzugt Metalloxiden, beträgt höchstens 7 Gew.-%, bevorzugt höchstens 5 Gew.-%.

Die Verunreinigungen umfassen insbesondere Metalle oder Metallverbindungen der Übergangsmetalle, insbesondere der VII. und VIII. Nebengruppe des Periodensystems der Elemente, oder der Alkalimetalle oder der Erdalkalimetalle oder Silizium oder Siliziumoxid, und insbesondere Metalle oder Metallverbindungen ausgewählt aus der Gruppe Aluminium, Magnesium, Titan, Zirkon, Natrium, Kalzium, Eisen, Nickel, Mangan, Molybdän und Kobalt.

Der Gehalt der Kohlenstoffnanoröhrchen an pyrolytisch abgeschiedenem Kohlenstoff beträgt bevorzugt höchstens 7 Gew.-%, insbesondere bevorzugt höchstens 2 Gew.-%, ganz besonders bevorzugt höchstens 1 Gew.-%.

Die neuen Kohlenstoffnanoröhrchen weisen in einer bevorzugten Ausführung im Querschnitt gesehen einen maximalen Außendurchmesser von bis zu 500 nm, vorzugsweise 10 bis 100 nm, besonders bevorzugt 15 bis 50 nm auf.

Der minimale Durchmesser ist typischer weise ca. 3 nm

Die neuen Kohlenstoffnanoröhrchen sind z. B. zugänglich aus den erfindungsgemäßen Kohlenstoffnanoröhrchenpulvern über an sich bekannte Mahl- und Dispergierverfahren. Dazu verwendbar sind beispielhaft alle Arten von Mühlen, Dissolvern und Dispergatoren, insbesondere diejenigen, die hohe Drücke und Schergeschwindigkeiten für hohe Energieeinträge ermöglichen, ebenso Ultraschallemitter, Walzwerke, Kalander, ein- und mehrwellige Schneckenextruder, um nur einige zu nennen.

Weiterer Gegenstand der Erfindung ist daher ein Verfahren zur Herstellung der neuen Kohlenstoffnanoröhrchen durch Deagglomeration der erfindungsgemäßen Kohlenstoffnanoröhrchenpulver insbesondere durch Mahlen oder Dispergieren unter einem Energieeintrag, der nötig ist, die Kohlenstoffnanoröhrchenpulver in einzelne Kohlenstoffnanoröhrchen aufzulösen.

Da die vereinzelten Kohlenstoffnanoröhrchen stark zur Agglomerierung neigen führt man die Deagglomeration bevorzugt in einer stabilisierenden Matrix aus z.B. in einem von sich aus noch flüssigen oder in einem verflüssigten Polymer.

Ein weiterer Gegenstand der Erfindung ist Verwendung der neuen Kohlenstoffnanoröhrchenpulver oder der Kohlenstoffnanoröhrchen als Zusatzstoff in anderen bekannten Matrixwerkstoffen, z.B. für Polymere, Kautschuke, Klebstoffe, Anstrichmittel, Dichtungsmassen, Keramik, Metalle, Metalllegierungen, Gläser, Beton und andere Baustoffe, Textilien und Verbundwerkstoffe oder als Adsorber insbesondere für flüchtige Verbindungen, z.B. für Gase oder für biologische Verbindungen, z.B. für Enzyme.

Gegenstand der Erfindung ist weiterhin die Verwendung der neuen Kohlenstoffnanoröhrchenpulver oder der Kohlenstoffnanoröhrchen als Bestandteil von Gasspeichermaterialien, insbesondere für die Speicherung von Wasserstoff.

Gegenstand der Erfindung ist ferner die Verwendung der neuen Kohlenstoffnanoröhrchenpulver oder der Kohlenstoffnanoröhrchen als leitfähiger Zusatz oder aktive bzw. Oberfläche vergrößernde Komponente in Elektroden, in Solarzellen, Aktuatoren, Sensoren, Tinten, oder Pasten sowie in Energiespeichern, insbesondere in Batterien, Kondensatoren (Supercaps) Brennstoffzellen oder Akkumulatoren,

Gegenstand der Erfindung ist ferner die Verwendung der neuen Kohlenstoffnanoröhrchenpulver oder der Kohlenstoffnanoröhrchen als Substrat für Katalysatoren.

Gegenstand der Erfindung ist auch noch die Verwendung der neuen Kohlenstoffnanoröhrchenpulver oder der Kohlenstoffnanoröhrchen als Substrat für pharmazeutische Wirkstoffe oder für Pflanzenschutz-Wirkstoffe

Die Erfindung wird nachstehend anhand der Figuren beispielhaft näher erläutert. Die Figuren 1 bis 3 zeigen schematisch verschiedene bekannte Strukturen von Kohlenstoffröhrchen und Figuren 4 und 5 Ansichten der erfmdungsgemäßen Kohlenstoffnanoröhrchen.

Im Einzelnen zeigen:

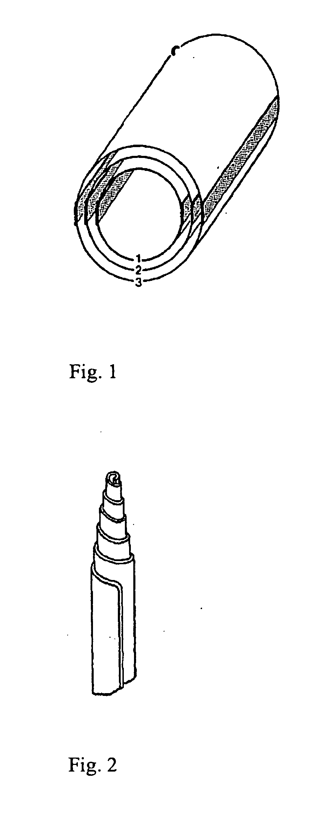

Fig. 1 mehrwandige zylindrische Kohlenstoffröhrchen (nach Iijima, Nature 354, 56-58, 1991),
Fig. 2 Kohlenstoffröhrchen mit Scroll-type Struktur nach Bacon (J. Appl. Phys. 34, 1960, 283-90)

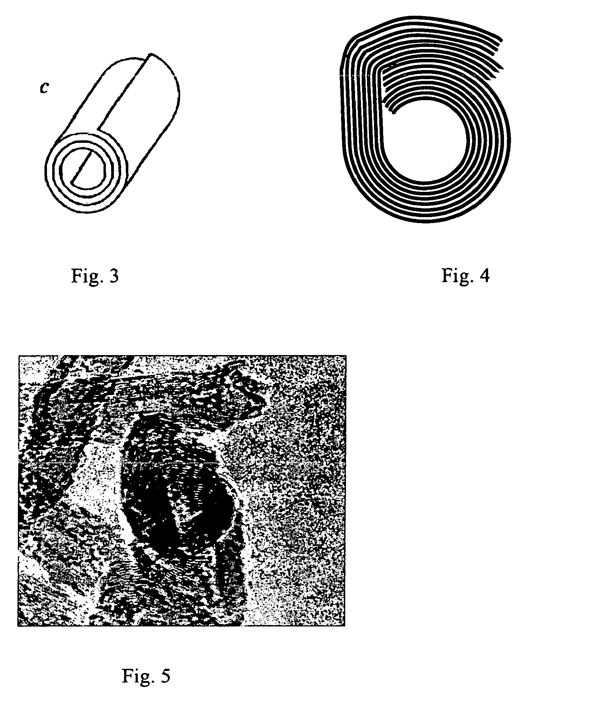

Fig. 3 die einfache Form der Kohlenstoffröhrchen mit Scroll-type Struktur nach Ijima (Nature 354, 56-58, 1991)
Fig. 4 schematisch den Aufbau der erfindungsgemäßen Kohlenstoffnanoröhrchen im Querschnitt
Fig. 5 den Querschnitt eines erfindungsgemäßen Kohlenstoffnanoröhrchens als Aufnahme im Transmissionselektronenmikroskop.

### Beispiele

### Beispiel 1: (Herstellung des Katalysators)

Eine Lösung von 0,306 kg Mg(NO₃)₂*6H₂O in Wasser (0,35 Liter) wurde mit einer Lösung von 0,36 kg Al(NO₃)₃*9H₂O in 0,351 Wasser vermischt. Anschließend wurden 0,17 kg Mn(NO₃)₂*4H₂O und 0,194 kg Co(NO₃)₂*6H₂O, jeweils gelöst in 0,51 Wasser hinzugegeben und die gesamte Mischung unter 30 min Rühren mittels Zugabe von Salpetersäure auf einen pH-Wert von ca. 2 gebracht. Ein Strom dieser Lösung wurde in einem Mischer mit 20,6 Gew.-%iger Natronlauge in einem Verhältnis von 1,9:1 verntischt und die entstehende Suspension in eine Vorlage von 51 Wasser gegeben. Der pH-Wert der Vorlage wurde durch Steuerung der Natronlaugezugabe auf ca. 10 gehalten.

Der ausgefallene Feststoff wurde von der Suspension abgetrennt und mehrfach gewaschen. Der gewaschene Feststoff wurde dann innerhalb von 16h in einem Schaufeltrockner getrocknet, wobei die Temperatur des Trockners innerhalb der ersten acht Stunden von Raumtemperatur auf 160 °C erhöht wurde. Danach wurde der Feststoff in einer Labormühle auf eine mittlere Teilchengröße von 50 µm gemahlen und die mittlere Fraktion im Bereich von 30 µm bis 100 µm Teilchengröße entnommen, um die nachfolgende Kalzinierung zu erleichtern, vor allem die Fluidisierung in der Wirbelschicht zu verbessern und eine hohe Ausbeute an Produkt zu erzielen. Anschließend wurde der Feststoff über 12 Stunden in einem Ofen von 500 °C unter Luftzugang kalziniert und dann über 24 Stunden abgekühlt. Das Katalysatormaterial wurde dann noch 7 Tage lang zur Nachoxidation bei Raumtemperatur stehen gelassen. Es wurden insgesamt 121,3 g Katalysatormaterial isoliert.

### Beispiel 2: (Herstellung der CNT in einer Wirbelschicht)

Der in Beispiel 1 hergestellte Katalysator wurde in einer Wirbelschichtapparatur im Labormaßstab getestet. Hierzu wurde eine definierte Menge an Katalysator in einem von außen durch einen Wärmeträger beheizten Stahlreaktor mit einem inneren Durchmesser von 100 mm vorgelegt. Die Temperatur der Wirbelbettes wurde über eine PID-Regelung des elektrisch beheizten Wärmeträgers geregelt. Die Temperatur des Wirbelbettes wurde durch ein Thenmoelement bestimmt. Eduktgase und inerte Verdünnungsgase wurden über elektronisch gesteuerte Massendurchflussregler in den Reaktor geleitet.

Der Reaktor wurde zunächst mit Stickstoff inertisiert und auf eine Temperatur von 650°C aufgeheizt. Dann wurde eine Menge von 24g Katalysator 1 gemäß Beispiel 1 eindosiert.

Danach wurde unmittelbar das Eduktgas als Mischung von Ethen und Stickstoff zugeschaltet. Das Volumenverhältnis der Eduktgasmischung betrug Ethen:N₂ = 90:10. Der Gesamtvolumenstrom wurde auf 40 LN-min⁻¹ eingestellt. Die Beaufschlagung des Katalysators mit den Eduktgasen erfolgte für einen Zeitraum von 33 Minuten. Danach wurde die laufende Reaktion durch Unterbrechung der Eduktzufuhr abgestoppt und der Reaktorinhalt entnommen.

Die Menge an abgeschiedenem Kohlenstoff wurde durch Auswiegen bestimmt und die Struktur und Morphologie des abgeschiedenen Kohlenstoffs wurde mit Hilfe von REM- und TEM-Analysen ermittelt. Die Menge an abgeschiedenem Kohlenstoff im Bezug auf eingesetzten Katalysator, im weiteren als Ertrag bezeichnet, wurde auf Basis der Masse an Katalysator nach Kalzinierung (mkat,0) und dem Gewichtszuwachs nach Reaktion (mgesamt-mkat,0) definiert: Ertrag = (mgesamt-mKat,0)/mKat,0.

Die Auswertung ergab eine über 5 Versuchsläufe gemittelte Katalysatorausbeute von 35,3 g Kohlenstoffnanoröhrchenpulver pro g eingesetztem Katalysator. In den TEM Aufnahmen zeigten sich Strukturen von ca. 2 bis3 aufgerollten Graphitschichten bestehend aus jeweils 8 bis 12 Graphenlagen. Die Kohlenstofffasern hatten einen mittleren Durchmesser von 16 nm.. Das Länge zu Durchmesser - Verhältnis betrug mindestens 100.

Die Überprüfung der Reinheit durch Glühverlust ergab einen Gehalt von 96,9 % Kohlenstoff.

Auf den TEM-Aufnahmen war kein pyrolytisch abgeschiedener Kohlenstoff in den Kohlenstoffnanoröhrchenpulvern zu erkennen.

Das Kohlenstoffnanoröhrehenpulver wies eine Oberfläche gemessen nach BET von 260 m²/g auf.

Die über 5 Versuchsläufe gemittelte Schüttdichte des Agglomerats betrug 152 kg/m³.

### Beispiel 3: (Vereinzelung der Kohlenstoffnanoröhrchen)

Aufgrund der hohen Oberfläche der Kohlenstoffnanoröhrchen ist eine Vereinzelung nur in Kombination mit einer Stabilisierung (Immobilisierung in einer Matrix, Zugabe von als Stabilisator wirkenden Stoffen) des Vereinzelungszustandes sinnvoll, da anderenfalls durch die hohen van der Waals Kräfte eine schnelle Reagglomeration der Kohlenstoffnanoröhrchen auftritt.

Das in Beispiel 2 erzeugte Kohlenstoffnanoröhrchenpulver wurde zusammen mit Polycarbonat (Makrolon 2805) in den Haupteinzug eines ko-rotierenden Doppelschneckenextruder (ZSK 26Mc, L/D 36) eingeführt. Die Temperatur des Extruders betrug 280°C. Der Durchsatz wurde auf 26kg/ h Komposit eingestellt. Die Drehzahl wurde auf 400 rpm eingestellt. Das Massenverhältnis des Kohlenstoffnanoröhrchenpulvers zu Polycarbonat betrug 5 : 95. Der aus dem Extruder austretende Strang wurde abgekühlt im Wasserbad und anschließend granuliert. Eine TEM Aufnahme eines von dem Komposit angefertigten Schnittes zeigt die Kohlenstoffnanoröhrchen vereinzelt im Polycarbonat vorliegen.

## Patentansprüche

1. Kohlenstoffnanoröhrchenpulver, aufweisend Kohlenstoffanoröhrchen, **dadurch gekennzeichnet, dass** die Kohlenstoffnanoröhrchen im wesentlichen aus einer oder mehreren Graphitschichten bestehen, wobei die Graphitschichten aus zwei oder mehr übereinander angeordneten Graphenschichten aufgebaut sind, und die Graphitschichten eine gerollte Struktur bilden, dass die Kohlenstoffnanoröhrchen im Querschnitt eine spiralförmige Anordnung der Graphitschichten aufweisen wobei der mittlere Durchmesser der Kohlenstoffnanoröhrchen 3 bis 100 nm, bevorzugt 4 bis 75 nm, besonders bevorzugt 5 bis 30 nm beträgt.

2. Kohlenstoffnanoröhrchenpulver nach Anspruch 1, **dadurch gekennzeichnet, dass** das Länge zu Durchmesser-Verhältnis der Kohlenstoffnanoröhrchen mindestens 5, insbesondere mindestens 30, besonders bevorzugt mindestens 50, beträgt.

3. Kohlenstoffnanoröhrchenpulver nach Anspruch 1 oder 2, **dadurch gekennzeichnet, dass** der Gehalt des Pulvers an Verunreinigungen, insbesondere von Metallen oder Metallverbindungen, insbesondere bevorzugt Metalloxiden, höchstens 7 Ges.-%, bevorzugt höchstens 5 Gew.-% beträgt.

4. Kohlenstoffnanoröhrchenpulver nach Anspruch 3, **dadurch gekennzeichnet, dass** die Verunreinigungen Metalle oder Metallverbindungen der Übergangsmetalle, insbesondere der VII. und VIII. Nebengruppe des Periodensystems der Elemente, oder der Alkalimetalle oder der Erdalkalimetalle oder Silizium oder Siliziumoxid umfassen, und insbesondere Metalle oder Metallverbindungen ausgewählt aus der Gruppe Aluminum, Magnesium, Titan, Zirkon, Natrium, Kalzium, Eisen, Nickel, Mangan, Molybdän und Kobalt umfassen.

5. Kohlenstoffnanoröhrchenpulver gemäß einem der Ansprüche 1 bis 4, **dadurch gekennzeichnet, dass** der Gehalt des Pulvers an pyrolytisch abgeschiedenem Kohlenstoff höchstens 7 Gew.%, bevorzugt höchstens 2 Gew.-%, besonders bevorzugt höchstens 1 Gew.-% beträgt.

6. Kohlenstoffnanoröhrchenpulver gemäß einem der Ansprüche 1 bis 5, **dadurch gekennzeichnet, dass** das Kohlenstoffnanoröhrchenpulver als Agglomerat vorliegt, wobei mindestens 95 Gew.% der Agglomeratteilchen einen Außendurchmesser im Bereich von 5 µm bis 10.000 µm, bevorzugt 30 µm bis 5.000 µm, besonders bevorzugt 50 µm bis 3.000 µm haben.

7. Kohlenstoffnanoröhrchenpulver nach einem der Ansprüche 1 bis 5, **dadurch gekennzeichnet, dass** die Schüttdichte (gemäß BN ISO 60) des Kohlenstoffnanoröhrchenpulvers 20 bis 450 kg/m³ , bevorzugt 80 bis 350 kg/m³, ganz besonders bevorzugt 110 bis 250 kg/m³ beträgt.

8. Kohlenstoffnanoröhrchenpulver gemäß einem, der Ansprüche 1 bis 7, **dadurch gekennzeichnet, dass** die spezifische Oberfläche (Stickstoffadsorption gemäß BET) 20 bis 1500 m²/g, bevorzugt 30 bis 800 m²/g besonders bevorzugt 90 bis 600 m²/g beträgt.

9. Kohlenstoffnanoröhrchen gemäß Anspruch 1.

10. Kohlenstoffnanoröhrchen nach Anspruch 9, **dadurch gekennzeichnet, dass** das Länge zu Durchmesser-Verhältnis der Kohlenstoffnanoröhrchen mindestens 5, insbesondere mindestens 10, insbesondere bevorzugt mindestens 20, ganz besonders Bevorzugt mindestens 50 beträgt.

11. Kohlenstoffnanoröhrchen nach Anspruch 9 oder 10, **dadurch gekennzeichnet, dass** der Gehalt der Kohlenstoffnanoröhrchen an Verunreinigungen insbesondere von Metallen oder Metallverbindungen, insbesondere bevorzugt Metalloxiden, höchstens 7 Gew.-%, bevorzugt böchstens 5 Gew.-% beträgt.

12. Kohlenstoffnanoröhrchen nach Anspruch 11, **dadurch gekennzeichnet, dass** die Verunreinigungen Metalle oder Metallverbindungen der Übergangsmetalle, insbesondere der VII. und VIII. Nebengruppe des Periodensystems der Elemente, oder der Alkalimetalle oder der Erdalkalimetalle oder Silizium oder Siliziumoxid umfassen, und insbesondere Metalle oder Metallverbindungen ausgewählt aus der Gruppe Aluminium, Magnesium, Titan, Zirkon, Natrium, Kalzium, Eisen, Nickel, Mangan, Molybdän und Kobalt umfassen.

13. Kohlenstoffnanoröhrchen gemäß einem der Ansprüche 9 bis 12, **dadurch gekennzeichnet, dass** der Gehalt der Kohlenstoffnanoröhrchen an Pyrolytisch abgeschiedenem Kohlenstoff höchstens 7 Gew.%, bevorzugt höchstens 2 Gew.%, besonders bevorzugt höchstens 1 Gew.-% beträgt.

14. Verfahren zur Herstellung eines Kohlenstoffnanoröhrchenpulvers nach einem der Ansprüche 1 bis 8, **dadurch gekennzeichnet, dass** C₁ - C₃-Kohlenwasserstoffe an einem heterogenen Katalysator bei einer Temperatur von 500 bis 1000°, bevorzugt 600 bis 800°C in einem Reaktor mit bewegtem Bett katalytisch zersetzt werden, wobei der verwendete Katalysator ein Übergangsmetallkatalysator basierend auf Co und Mn ist, mit einem Anteil von Co von 40 bis 60 mol.-% und von Mn 60 bis 40 mol.-% bezogen, auf die Summe von Co und Mn , die mittlere Verweilzeit des Katalysators in der Reaktionszone höchstens eine Stunde beträgt und der Kohlenwasserstoff einzeln, oder als Gemisch auch zusammen mit Inertgasen, insbesondere Stickstoff oder Edelgasen umgesetzt wird.

15. Verfahren nach Anspruch 14, **dadurch gekennzeichnet, dass** die mittlere Verweilzeit des Katalysators in der Reaktionszone 20 bis 45 min, bevorzugt 25 bis 35 min beträgt.

16. Verfahren nach Anspruch 15, **dadurch gekennzeichnet, dass** der Katalysator einen Anteil von Co von 45 bis 55 mol.% und von Mn von 55 bis 45 mol.% bezogen auf die Summe von Co und Mn aufweist

17. Verfahren zur Herstellung von Kohlenstoffhanoröhrchen nach einem der Ansprüche 9 bis 13, **dadurch gekennzeichnet, dass** Kohlenstoffnanoröhrchenpulver nach einem der Ansprüche 1 bis 8, insbesondere durch Mahlen oder Dispergieren, unter Energieeintrag deagglomeriert wird

18. Verwendung der Kohlenstoffnanohrchenpulver gemäß einem der Ansprüche 1 bis 8 oder der Kohlenstoffnanoröhrchen gemäß einem der Ansprüche 9 bis 13 als Zusatz für Polymer, Kautschuk, Keramik, Metalle, Metalllegierungen, Gläser, Textilien und Verbundwerkstoffe.

19. Verwendung der Kohlenstofffhanoröhrchenpulver gemäß einem der Ansprüche 1 bis 8 oder der Kohlenstoffnanoröhrchen gemäß einem der Ansprüche 9 bis 13 als leitfähiger Zusatz in Elektroden, Solarzellen, Aktuatoren, Sensoren, Tinten, oder Pasten sowie in Energiespeichern, insbesondere in Batterien, Akkumulatoren, Brennstoffzellen oder Kondensatoren; als Substrat für pharmazeutische Wirkstoffe oder für Pflanzenschutz-Wirkstoffe; als Adsorber insbesondere für flüchtige Verbindungen, z.B. für Gase oder für biologische Verbindungen, z.B. für Enzyme; oder als Träger oder Container von Katalysatoren.

## Claims

1. Carbon nanotube powder comprising carbon nanotubes, **characterized in that** the carbon nanotubes consist essentially of one or more graphite layers, said graphite layers being formed from two or more graphene layers arranged one on top of another, and said graphite layers forming a rolled structure such that the carbon nanotubes in cross section have a spiral arrangement of the graphite layers, the mean diameter of said carbon nanotubes being 3 to 100 nm, preferably 4 to 75 nm, more preferably 5 to 30 nm.

2. Carbon nanotube powder according to Claim 1, **characterized in that** the length to diameter ratio of the carbon nanotubes is at least 5, especially at least 30, more preferably at least 50.

3. Carbon nanotube powder according to Claim 1 or 2, **characterized in that** the content of impurities in the powder, especially of metals or metal compounds, especially preferably metal oxides, is at most 7% by weight, preferably at most 5% by weight.

4. Carbon nanotube powder according to Claim 3, **characterized in that** the impurities comprise metals or metal compounds of the transition metals, especially of transition groups VII and VIII of the Periodic Table of the Elements, or of the alkali metals or of the alkaline earth metals or silicon or silicon oxide, and especially metals or metal compounds selected from the group of aluminium, magnesium, titanium, zirconium, sodium, calcium, iron, nickel, manganese, molybdenum and cobalt.

5. Carbon nanotube powder according to any of Claims 1 to 4, **characterized in that** the content of pyrolytically deposited carbon in the powder is at most 7% by weight, preferably at most 2% by weight, more preferably at most 1% by weight.

6. Carbon nanotube powder according to any of Claims 1 to 5, **characterized in that** the carbon nanotube powder is in the form of an agglomerate, and at least 95% by weight of the agglomerate particles having an external diameter in the range from 5 µm to 10 000 µm, preferably 30 µm to 5000 µm, more preferably 50 µm to 3000 µm.

7. Carbon nanotube powder according to any of Claims 1 to 5, **characterized in that** the bulk density (to EN ISO 60) of the carbon nanotube powder is 20 to 450 kg/m³, preferably 80 to 350 kg/m³, most preferably 110 to 250 kg/m³.

8. Carbon nanotube powder according to any of Claims 1 to 7, **characterized in that** the specific surface area (BET nitrogen adsorption) is 20 to 1500 m²/g, preferably 30 to 800 m²/g, more preferably 90 to 600 m²/g.

9. Carbon nanotubes according to Claim 1.

10. Carbon nanotubes according to Claim 9, **characterized in that** the length to diameter ratio of the carbon nanotubes is at least 5, especially at least 10, more preferably at least 20, most preferably at least 50.

11. Carbon nanotubes according to Claim 9 or 10, **characterized in that** the content of impurities in the carbon nanotube powder, especially of metals or metal compounds, especially preferably metal oxides, is at most 7% by weight, preferably at most 5% by weight.

12. Carbon nanotubes according to Claim 11, **characterized in that** the impurities comprise metals or metal compounds of the transition metals, especially of transition groups VII and VIII of the Periodic Table of the Elements, or of the alkali metals or of the alkaline earth metals or silicon or silicon oxide, and especially metals or metal compounds selected from the group of aluminium, magnesium, titanium, zirconium, sodium, calcium, iron, nickel, manganese, molybdenum and cobalt.

13. Carbon nanotubes according to any of Claims 9 to 12, **characterized in that** the content of pyrolytically deposited carbon in the carbon nanotube powder is at most 7% by weight, preferably at most 2% by weight, more preferably at most 1% by weight.

14. Process for producing a carbon nanotube powder according to any of Claims 1 to 8, **characterized in that** C₁-C₃ hydrocarbons are catalytically decomposed over a heterogeneous catalyst at a temperature of 500 to 1000°C, preferably 600 to 800°C, in a reactor with a moving bed, the catalyst used being a transition metal catalyst based on Co and Mn, with a proportion of Co of 40 to 60 mol% and of Mn of 60 to 40 mol%, based on the sum of Co and Mn, the mean residence time of the catalyst in the reaction zone being at most one hour, and the hydrocarbon being reacted individually or as a mixture, including a mixture together with inert gases, especially nitrogen or noble gases.

15. Process according to Claim 14, **characterized in that** the mean residence time of the catalyst in the reaction zone is 20 to 45 min, preferably 25 to 35 min.

16. Process according to Claim 15, **characterized in that** the catalyst has a proportion of Co of 45 to 55 mol% and of Mn of 55 to 45 mol%, based on the sum of Co and Mn.

17. Process for producing carbon nanotubes according to any of Claims 9 to 13, **characterized in that** carbon nanotube powder according to any of Claims 1 to 8 is deagglomerated with introduction of energy, especially by grinding or dispersion.

18. Use of the carbon nanotube powder according to any of Claims 1 to 8 or of the carbon nanotubes according to any of Claims 9 to 13 as an additive for polymers, rubber, ceramic, metals, metal alloys, glasses, textiles and composite materials.

19. Use of the carbon nanotube powder according to any of Claims 1 to 8 or of the carbon nanotubes according to any of Claims 9 to 13 as a conductive additive in electrodes, solar cells, actuators, sensors, inks or pastes, and in energy stores, especially in batteries, accumulators, fuel cells or capacitors; as a substrate for active pharmaceutical ingredients or for active crop protection ingredients; as an adsorbent, especially for volatile compounds, for example for gases or for biological compounds, for example for enzymes, or as a support or container for catalysts.

## Revendications

1. Poudre de nanotubes de carbone présentant des nanotubes de carbone, **caractérisée en ce que** les nanotubes de carbone se composent essentiellement d'une ou de plusieurs couches de graphite, dans laquelle les couches de graphite sont construites à partir de deux ou plusieurs couches de graphènes disposées les unes au-dessus des autres, et les couches de graphite forment une structure enroulée, **en ce que** les nanotubes de carbone présentent en section transversale une disposition spiralée des couches de graphite, dans laquelle le diamètre moyen des nanotubes de carbone vaut 3 à 100 nm, de préférence 4 à 75 nm, et de préférence encore 5 à 30 nm.

2. Poudre de nanotubes de carbone selon la revendication 1, **caractérisée en ce que** le rapport de la longueur au diamètre des nanotubes de carbone vaut au moins 5, en particulier au moins 30, et en particulier de préférence au moins 50.

3. Poudre de nanotubes de carbone selon la revendication 1 ou 2, **caractérisée en ce que** la teneur de la poudre en impuretés, en particulier de métaux ou de composés métalliques, en particulier de préférence d'oxydes métalliques, vaut au maximum 7 % en poids, de préférence au maximum 5 % en poids.

4. Poudre de nanotubes de carbone selon la revendication 3, **caractérisée en ce que** les impuretés comprennent des métaux ou des composés métalliques des métaux de transition, en particulier des sous-groupes VII et VIII du tableau périodique des éléments, ou des métaux alcalins ou des métaux alcalino-terreux ou du silicium ou de l'oxyde de silicium, et comprennent en particulier des métaux ou des composés métalliques sélectionnés dans le groupe comprenant l'aluminium, le magnésium, le titane, le zirconium, le sodium, le calcium, le fer, le nickel, le manganèse, le molybdène et le cobalt.

5. Poudre de nanotubes de carbone selon l'une quelconque des revendications 1 à 4, **caractérisée en ce que** la teneur de la poudre en carbone précipité par pyrolyse vaut au maximum 7 % en poids, de préférence au maximum 2 % en poids, et en particulier de préférence au maximum 1 % en poids.

6. Poudre de nanotubes de carbone selon l'une quelconque des revendications 1 à 5, **caractérisée en ce que** la poudre de nanotubes de carbone se présente sous la forme d'un aggloméré, dans laquelle au moins 95 % en poids des particules de l'aggloméré possèdent un diamètre extérieur de l'ordre de 5 µm à 10 000 µm, de préférence de 30 µm à 5 000 µm, et en particulier de préférence de 50 µm à 3 000 µm.

7. Poudre de nanotubes de carbone selon l'une quelconque des revendications 1 à 5, **caractérisée en ce que** la densité en vrac (selon la norme EN ISO 60) de la poudre de nanotubes de carbone vaut de 20 à 450 kg/m³, de préférence 80 à 350 kg/m³, et tout particulièrement de préférence de 110 à 250 kg/m³.

8. Poudre de nanotubes de carbone selon l'une quelconque des revendications 1 à 7, **caractérisée en ce que** la surface spécifique (adsorption d'azote selon BET) vaut de 20 à 1 500 m²/g, de préférence de 30 à 800 m²/g, en particulier de préférence de 90 à 600 m²/g.

9. Nanotubes de carbone selon la revendication 1.

10. Nanotubes de carbone selon la revendication 9, **caractérisés en ce que** le rapport de la longueur au diamètre des nanotubes de carbone vaut au moins 5, en particulier au moins 10, en particulier de préférence au moins 20, et tout particulièrement de préférence au moins 50.

11. Nanotubes de carbone selon la revendication 9 ou 10, **caractérisés en ce que** la teneur des nanotubes de carbone en impuretés, en particulier de métaux ou de composés métalliques, en particulier de préférence d'oxydes métalliques, vaut au maximum 7 % en poids, de préférence au maximum 5 % en poids.

12. Nanotubes de carbone selon la revendication 11, **caractérisés en ce que** les impuretés comprennent des métaux ou des composés métalliques des métaux de transition, en particulier des sous-groupes VII et VIII du tableau périodique des éléments, ou des métaux alcalins ou des métaux alcalino-terreux ou du silicium ou de l'oxyde de silicium, et comprennent en particulier des métaux ou des composés métalliques sélectionnés dans le groupe comprenant l'aluminium, le magnésium, le titane, le zirconium, le sodium, le calcium, le fer, le nickel, le manganèse, le molybdène et le cobalt.

13. Nanotubes de carbone selon l'une quelconque des revendications 9 à 12, **caractérisés en ce que** la teneur des nanotubes de carbone en carbone précipité par pyrolyse vaut au maximum 7 % en poids, de préférence au maximum 2 % en poids, et en particulier de préférence au maximum 1 % en poids.

14. Procédé pour la production d'une poudre de nanotubes de carbone selon l'une quelconque des revendications 1 à 8, **caractérisé en ce que** l'on décompose de façon catalytique des hydrocarbures C₁-C₃ sur un catalyseur hétérogène à une température de 500 à 1000°C, de préférence de 600 à 800°C, dans un réacteur à lit fluidisé, dans lequel le catalyseur utilisé est un catalyseur de métaux de transition à base de Co et Mn, avec une proportion de Co de 40 à 60 % molaires et de Mn de 60 à 40 % molaires rapportés à la somme de Co et Mn, le temps de résidence moyen du catalyseur dans la zone de réaction est au maximum d'une heure et l'hydrocarbure est décomposé individuellement ou en mélange aussi avec des gaz inertes, en particulier de l'azote ou des gaz rares.

15. Procédé selon la revendication 14, **caractérisé en ce que** le temps de résidence moyen du catalyseur dans la zone de réaction est de 20 à 45 minutes, de préférence de 25 à 35 minutes.

16. Procédé selon la revendication 15, **caractérisé en ce que** le catalyseur présente une proportion de Co de 45 à 55 % molaires et de Mn de 55 à 45 % molaires, rapportés à la somme de Co et Mn.

17. Procédé pour la production de nanotubes de carbone selon l'une quelconque des revendications 9 à 13, **caractérisé en ce que** l'on désagrège une poudre de nanotubes de carbone selon l'une quelconque des revendications 1 à 8, en particulier par broyage ou dispersion, avec apport d'énergie.

18. Utilisation des poudres de nanotubes de carbone selon l'une quelconque des revendications 1 à 8 ou des nanotubes de carbone selon l'une quelconque des revendications 9 à 13 comme additifs pour des polymères, du caoutchouc, des céramiques, des métaux, des alliages métalliques, des verres, des textiles et des matériaux composites.

19. Utilisation des poudres de nanotubes de carbone selon l'une quelconque des revendications 1 à 8 ou des nanotubes de carbone selon l'une quelconque des revendications 9 à 13 comme additif conducteur dans des électrodes, des cellules solaires, des actionneurs, des capteurs, des encres, ou des pâtes ainsi que dans des accumulateurs d'énergie, en particulier dans des batteries, des accumulateurs, des piles à combustible ou des condensateurs; comme substrat pour des substances actives pharmaceutiques ou pour des substances actives de protection végétale; comme adsorbeur en particulier pour des composés volatils, par exemple pour des gaz ou pour des composés biologiques, par exemple pour des enzymes; ou comme support ou boîtier de catalyseurs.
